(19) **Europäisches Patentamt / European Patent Office / Office européen des brevets**

(11) **EP 4 748 235 A1**

(12) # EUROPEAN PATENT APPLICATION
published in accordance with Art. 153(4) EPC

(43) Date of publication:
**27.05.2026 Bulletin 2026/22**

(21) Application number: **24845543.8**

(22) Date of filing: **19.07.2024**

(51) International Patent Classification (IPC):
**A23C 11/10** (2025.01)     **A23L 5/00** (2016.01)
**A23L 31/15** (2016.01)

(52) Cooperative Patent Classification (CPC):
**A23C 11/00; A23C 11/10; A23L 5/00; A23L 11/60; A23L 11/65; A23L 29/00; A23L 31/10; A23L 31/15; C12N 9/24**

(86) International application number:
**PCT/JP2024/025900**

(87) International publication number:
**WO 2025/023166 (30.01.2025 Gazette 2025/05)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **21.07.2023 JP 2023119306**

(71) Applicant: **Asahi Group Holdings, Ltd.**
**Tokyo 130-8602 (JP)**

(72) Inventors:
- **OTSUSHI, Noboru**
  **Moriya-shi, Ibaraki 302-0106 (JP)**
- **KATO, Yuki**
  **Moriya-shi, Ibaraki 302-0106 (JP)**

(74) Representative: **J A Kemp LLP**
**80 Turnmill Street**
**London EC1M 5QU (GB)**

(54) **MILK SUBSTITUTE COMPOSITION CONTAINING YEAST EXTRACT FUNGAL RESIDUE**

(57) This is to provide a milk-substitute composition for the purpose of protein supplementation by reducing the characteristic flavor (umami and sourness) derived from yeast materials.

This is to provide a milk-substitute composition comprising a yeast cell body residue or its cell wall lysing enzyme-decomposition product, characterized in that a total amount of free amino acids (in particular, glutamic acid) contained in the residue or the decomposition product is less than a predetermined amount, and a milk-substitute food and drink containing the composition. In the composition of the present invention, the characteristic flavor peculiar to yeast is markedly reduced, so that it can be formulated as a milk-substitute composition in various foods and drinks with a higher concentration than conventional products.

**Description**

TECHNICAL FIELD

The present invention relates to a milk-substitute composition containing a yeast cell body residue or a cell wall lysing enzyme-decomposition product thereof, characterized in that the total amount of free amino acids (in particular, glutamic acid) contained in the residue or the decomposition product is less than a predetermined amount, and to a milk-substitute food and drink containing the composition.

BACKGROUND ART

In recent years, in order to avoid allergens derived from milk, from an increase in demand for low-fat or non-fat foods due to diet consciousness and health consciousness, or from an increase in demand for vegan foods, etc., more attention has been paid to foods using a milk-like substitute derived from plants such as oats, soybeans, almonds, etc., in place of a milk raw material. However, these milk-like substitutes derived from plants are usually more expensive than cow's milk and are known to be foods containing allergic substances.

In addition to such a milk-like substitute derived from a plant, a milk-like substitute derived from a yeast material has also been reported. For example, the present applicant has reported use of a composition containing a yeast cell wall decomposition product, which contains a cell wall lysing enzyme-decomposition product of residues (yeast cell body residue) after production of yeast extract, as a milk-like substitute (for example, see Patent Document 1).

PRIOR ART DOCUMENT

PATENT DOCUMENT

Patent Document 1: JP 2022-135934A

SUMMARY OF THE INVENTION

PROBLEMS TO BE SOLVED BY THE INVENTION

However, milk-like substitutes derived from yeast materials have a characteristic flavor (umami, sourness). Therefore, for the purpose of supplementing proteins, when milk-like substitutes derived from yeast materials were used at a concentration of about 5%, there is a problem that it is not suitable for direct drinking due to the strong flavor.

MEANS TO SOLVE THE PROBLEMS

The present inventors have earnestly studied to solve the above-mentioned problems, and as a result, they have found that, in a milk-substitute composition comprising a yeast cell body residue or a cell wall lysing enzyme-decomposition product thereof, the characteristic flavor is reduced by making the total amount of free amino acids (in particular, glutamic acid) and acid components (in particular, phosphoric acid), etc., contained in the residue or the decomposition product less than predetermined amounts, and it can be directly applied to drink as a milk-substitute composition, whereby they have completed the present invention.

Accordingly, the gist of the present invention is as follows.

[1] A milk-substitute composition comprising a yeast cell body residue, wherein a total amount of free glutamic acid contained in the residue is less than 0.5% by mass based on a dry mass of the residue.

[2] The composition described in [1], wherein a total amount of free amino acids contained in the residue is less than 1% by mass based on a dry mass of the residue.

[3] The composition described in [1] or [2], wherein a total amount of phosphoric acid contained in the residue is less than 0.1% by mass based on a dry mass of the residue.

[4] The composition described in any one of [1] to [3], wherein the residue is a water-insoluble fraction after hot water extraction of yeast.

[5] The composition described in any one of [1] to [4], wherein the soluble solid content contained in the residue is less than 5% by mass based on a dry mass of the residue.

[6] A milk-substitute composition comprising a cell wall lysing enzyme-decomposition product of a yeast cell body residue, wherein a total amount of free glutamic acid contained in the decomposition product is less than 0.4% by mass based on a dry mass of the decomposition product.

[7] The composition described in [6], wherein a total amount of free amino acids contained in the decomposition product is less than 1% by mass based on a dry mass of the decomposition product.

[8] The composition described in [6] or [7], wherein a total amount of phosphoric acid contained in the decomposition product is less than 0.20% by mass based on a dry mass of the decomposition product.

[9] The milk-substitute composition comprising a cell wall lysing enzyme-decomposition product of a yeast cell body residue described in any one of [6] to [8], wherein a soluble solid content contained in the residue is less than 5% by mass based on a dry mass of the residue.

[10] The composition described in any one of [6] to [9], wherein the decomposition product is a material obtained by a method which comprises the following:

(a) treating the yeast cell body residue with a cell wall lysing enzyme at 40 to 60°C for 1 to 24 hours, and
(b) collecting the treated product obtained in (a).

[11] The composition described in any one of [6] to [10], wherein the cell wall lysing enzyme is a glucanase.

[12] The composition described in any one of [6] to [11], wherein the cell wall lysing enzyme is a glucanase having β-1,3, β-1,4, and/or β-1,6 activity.

[13] The composition described in any one of [6] to [12], wherein the cell wall lysing enzyme is a glucanase derived from *Streptomyces.*

[14] The composition described in any one of [6] to [13], wherein the cell wall lysing enzyme is a glucanase having no protease activity.

[15] A milk-substitute food and drink comprising the composition described in any of [1] to [14].

[16] The milk-substitute food and drink described in [15], which further comprises 0.1 to 30% by mass of oils and fats.

[17] A method for improving flavor of a milk-substitute composition comprising a yeast cell body residue, which comprises reducing a total amount of free glutamic acid contained in the residue to less than 0.5% by mass based on a dry mass of the residue.

[18] A method for improving flavor of a milk-substitute composition comprising a cell wall lysing enzyme-decomposition product of a yeast cell body residue, which comprises reducing a total amount of free glutamic acid contained in the decomposition product to less than 0.4% by mass based on a dry mass of the decomposition product.

[19] Use of a yeast cell body residue as a milk-substitute composition, wherein a total amount of free glutamic acid contained in the residue is less than 0.5% by mass based on a dry mass of the residue.

[20] Use of a cell wall lysing enzyme-decomposition product of a yeast cell body residue as a milk-substitute composition, wherein a total amount of free glutamic acid contained in the decomposition product is less than 0.4% by mass based on a dry mass of the decomposition product.

EFFECTS OF THE INVENTION

According to the present invention, it is possible to provide a milk-substitute composition using inexpensive and easily available yeast cell body residue as a raw material. In the composition of the present invention, by controlling a total amount of free amino acids (in particular, glutamic acid) and acid components (in particular, phosphoric acid), etc., contained in a yeast cell body residue or a cell wall lysing enzyme-decomposition product thereof to less than a specific amount, the characteristic flavor (in particular, umami and sourness) peculiar to yeast is markedly reduced, and thus it can be formulated in various foods and drinks as a milk-substitute composition in higher concentrations than conventional products. Therefore, according to the composition of the present invention, intake of yeast-derived protein, dietary fiber, and other nutrients, etc., can be easily carried out.

BRIEF DESCRIPTION OF THE DRAWINGS

Fig. 1 is a radar chart showing the results (average values) of the sensory evaluation of five expert panelists for the Formulations 1 and 2 in Test Example 2.

Fig. 2 is a radar chart showing the results (average values) of the sensory evaluation of five expert panelists for the Formulations 3 and 4 in Test Example 2.

Fig. 3 is a radar chart showing the results (average values) of the sensory evaluation of five expert panelists for the Formulations 5 and 6 in Test Example 2.

Fig. 4 is a radar chart showing the results (average values) of the sensory evaluation of five expert panelists for the Formulations 7 and 8 in Test Example 2.

Fig. 5 is a radar chart showing the results (average values) of the sensory evaluation of five expert panelists for the Formulations 9 and 10 in Test Example 2.
Fig. 6 is a radar chart showing the results (average values) of the sensory evaluation of five expert panelists for the Formulations 11 and 12 in Test Example 2.
Fig. 7 is a radar chart showing the results (average values) of the sensory evaluation of five expert panelists for the yeast cell body residue (compositions of Comparative Examples 1, 3 and 4, and Examples 1 and 3) in Test Example 5.
Fig. 8 is a radar chart showing the results (average values) of the sensory evaluation of five expert panelists for the cell wall lysing enzyme decomposition products (compositions of Comparative Examples 2, 5 and 6 and Examples 2 and 4) in Test Example 5.

EMBODIMENTS TO CARRY OUT THE INVENTION

In one embodiment of the present invention, it is preferably provided a milk-substitute composition containing a yeast cell body residue, wherein the total amount of free glutamic acid contained in the residue is less than 0.5% by mass based on the dry mass of the residue.

In the present invention, the terms "milk-substitute composition" mean a composition that can be used in place of milk (typically, cow's milk) that humans eat and drink. Milk that humans eat and drink is a cloudy white liquid secreted by the mammary glands of mammals with nutrients such as water, protein, fat, carbohydrates, vitamins, and minerals, etc., and having a distinctive milk flavor, and the milk-substitute composition means a liquid that is to eat and drink having one or a plurality of these characteristics. As the milk-substitute composition, there are those generally produced from vegetable raw materials such as soybeans, almonds, oats, rice, coconut, etc., containing proteins and lipids derived from plants, and containing dietary fiber, which is rarely contained in milk. There is a case where those produced from these vegetable raw materials are used on their own or may be used by mixing several kinds, or there is a case where it is used by mixing with milk derived from animals. The milk-substitute composition of the present invention may also be used in place of such known milk-substitute compositions produced from such known vegetable raw materials.

The terms "yeast cell body residue" used in in the present specification are not particularly limited as long as it is a material containing cell wall of yeast and is a residue (insoluble fraction) of yeast cell body obtained after yeast is subjected to extraction treatment. Specifically, it means the yeast cell body generating as a residue by subjecting yeast to known extraction treatments such as self-digestion treatment (protease treatment), hot water treatment, acid treatment, alkali treatment, and/or mechanical pulverization treatment, etc., and removing the supernatant (water-soluble fraction (yeast extract)) separated by centrifugation etc. The yeast cell body residue is preferably a residue (water insoluble fraction) of the yeast cell body after extraction of yeast with hot water. The yeast cell body residue (water insoluble fraction) according to the present invention is preferably containing the soluble solid content of less than 5% by mass based on the dry mass. Soluble of the soluble solids means soluble in water, and the soluble solid is calculated as a ratio (% by mass) occupied in the dry mass of the yeast cell body residue. The content of the soluble solids contained in the yeast cell body residue according to the present invention can be measured, for example, according to the method described in Test Example 4 mentioned hereinbelow.

Also, the terms "dry mass of the yeast cell body residue" used in the present specification mean the mass of the yeast cell body residue after drying the yeast cell body residue by known methods. The drying method is not particularly limited and a known drying method can be used, and an atmospheric heating drying, vacuum drying, spray drying, freeze drying, etc., can be used. For example, it can be obtained by drying the sample at 105°C for 5 hours in an atmospheric heating dryer and weighing the residue.

The yeast cell body residue is constituted by protein, lipids, ash, and dietary fiber, etc. The contained ratio of the protein is, for example, from 20% by mass to 60% by mass based on the dry mass of the yeast cell body residue. The contained ratio of the lipids is, for example, from 1% by mass to 10% by mass based on the dry mass of the yeast cell body residue. The contained ratio of the ash is, for example, from 0% by mass to 10% by mass based on the dry mass of the yeast cell body residue. The contained ratio of the dietary fiber is, for example, from 10% by mass to 60% by mass based on the dry mass of the yeast cell body residue. In addition to the general nutritional components, components which are reported to have physiological functions, such as $\beta$-glucan and $\alpha$-mannan are contained in the yeast cell body residue. $\beta$-Glucan is a polysaccharide in which D-glucose is polymerized by $\beta$-1,3 bond and $\beta$-1,6 bond, and is classified as a dietary fiber since it is a difficultly digestible component in foods that cannot be digested by human digestive enzymes. $\alpha$-Mannan is a polysaccharide in which D-mannose is polymerized by $\alpha$-1,6 bond, $\alpha$-1,2 bond or $\alpha$-1,3 bond, and is classified as a dietary fiber since it is a difficultly digestible component in foods that cannot be digested by human digestive enzymes. The contained ratio of the $\beta$-glucan is, for example, from 10% by mass to 40% by mass based on the dry mass of the yeast cell body residue. The contained ratio of the $\alpha$-mannan is, for example, from 10% by mass to 40% by mass based on the dry mass of the yeast cell body residue. The content of each component can be determined by known analytical method. The content of the protein can be obtained, for example, by measuring as an amount of nitrogen by the Kjeldahl method and multiplying the amount of nitrogen by a conversion factor of 6.25. The content of the lipids can be determined, for example, by the acid decomposition method. The content of the ash can be determined by the direct ashing method. The content of the dietary fiber can be determined, for example, by the Prosky method (enzyme-gravimetric method) or by high-performance liquid chromatography (enzyme-HPLC method). The content of the $\alpha$-mannan can be measured, for example, by determining mannose formed by hydrolyzing mannan. The content of the $\beta$-glucan can be measured, for example, by using a (1-3), (1-4)-$\beta$-glucan assay kit (manufactured by Megazyme Ltd.).

In the residue (water insoluble fraction) of the yeast cell body obtained in this way, when the total amount of free amino acids (in particular, glutamic acid) and acid components is the predetermined amount mentioned below or more, by subjecting to appropriate purification and washing operation depending on necessity, the yeast cell body residue according to the present invention, in which the total amount of the free amino acids (in particular, glutamic acid) and the acid components is less than the predetermined amount mentioned below can be obtained. As such purification and washing operation, there may be mentioned, for example, repeated extraction and separation processes and increased amounts of washing water, etc.

As the yeast, it is not particularly limited as long as it can be applied to food field, and there may be mentioned, for example, yeast for beer production, yeast for bread production, yeast for rice wine production, etc. Alternatively, as the yeast, whereas it is not limited to these, there may be mentioned, for example, those belonging to the genera *Saccharomyces, Saccharomycoides, Rhodotorula, Endomycopsis, Nematospora, Brettanomyces, Candida, Torulopsis,* etc. Among these, it is preferably *Saccharomyces cerevisiae, Saccharomyces pastorianus,* and *Saccharomyces bayanus.* These may be alone or may be a combination of two or more kinds.

The yeast cell body residue according to the present invention is characterized in that the total amount of free glutamic acid is reduced to less than a predetermined amount. Specifically, the total amount of free glutamic acid contained in the yeast cell body residue according to the present invention is less than 0.5% by mass, preferably less than 0.4% by mass, and more preferably less than 0.3% by mass based on the dry mass of the residue. It has been known that glutamic acid is an acidic amino acid and contributes to sourness and its sodium salt has strong umami, and the present inventors have found in the yeast cell body residue that the characteristic flavor peculiar to the yeast is markedly reduced by controlling the content thereof to less than 0.5% by mass.

In addition, the yeast cell body residue according to the present invention is preferably one in which the total amount of free amino acids as well as free glutamic acid is reduced to less than a predetermined amount. Specifically, it is preferable that the total amount of free amino acids contained in the yeast cell body residue according to the present invention is less than 1% by mass based on the dry mass of the residue, more preferably less than 0.8% by mass, and further preferably less than 0.5% by mass.

Incidentally, in the present invention, the "total amount of free amino acids" means a total of each amount of histidine (His), asparagine (Asn), serine (Ser), glutamine (Gln), arginine (Arg), glycine (Gly), aspartic acid (Asp), glutamic acid (Glu), threonine (Thr), alanine (Ala), $\gamma$-aminobutyric acid (GABA), proline (Pro), cysteine (Cys), lysine (Lys), tyrosine (Tyr), methionine (Met), valine (Val), isoleucine (Ile), leucine (Leu), phenylalanine (Phe) and tryptophan (Trp) as measured by UPLC analysis (labeling method) in Examples mentioned later and, for example, as described in Examples mentioned later, it can be calculated from each measured value obtained by UPLC analysis (labeling method). Incidentally, the free amino acids may be either of L-isomer, D-isomer and DL-isomer, and preferably L-isomer.

Also, it has been known that amino acids are involved in bitter taste and sweet taste, etc. Accordingly, in the yeast cell body residue according to the present invention, it is preferable that the total amount of the free amino acids which have been known to exhibit bitter taste and/or sweet taste is also reduced to less than the predetermined amount. Accordingly, for example, in the yeast cell body residue according to the present invention, the total amount of phenylalanine, tyrosine, arginine, isoleucine, leucine, valine, methionine and lysine which are free amino acids and which have been known to exhibit bitter taste, is preferably less than 0.07% by mass based on the dry mass of the residue, more preferably less than 0.05% by mass, and further preferably less than 0.03% by mass. Also, the total amount of alanine and proline which are free amino acids and which have been known to exhibit sweet taste, is preferably less than 0.2% by mass based on the dry mass of the residue, more preferably less than 0.1% by mass, and further preferably less than 0.05% by mass. In the yeast cell body residue according to the present invention, by controlling the total amount of the free amino acids which have been known to exhibit bitter taste or sweet taste, further reduction of the characteristic flavor peculiar to yeast can be expected.

The yeast cell body residue according to the present invention is also preferably one in which the total amount of the acid components is reduced to less than a predetermined amount. Specifically, the acid components contained in the yeast cell body residue according to the present invention are typically phosphoric acid and citric acid, and the content of the phosphoric acid is preferably less than 0.1% by mass based on the dry mass of the residue, and more preferably less than 0.05% by mass. Also, the content of the citric acid is preferably less than 0.03% by mass based on the dry mass of the residue, and more preferably less than 0.02% by mass. In the yeast cell body residue according to the present invention, by controlling the total amount of the acid components, further reduction of the characteristic flavor peculiar to yeast can be expected.

The milk-substitute composition containing the yeast cell body residue of the present invention may be in the form of a cloudy liquid or paste containing an appropriate concentration of the yeast cell body residue in a suitable medium (preferably water) containing, for example, about 0.001 to about 30% by mass, and preferably about 5 to about 20% by mass. Alternatively, the milk-substitute composition of the present invention may be the yeast cell body residue (water insoluble fraction) itself, preferably in the form of a powder with a soluble solid content of less than 5% by mass. By utilizing the composition of the present invention, nutrients such as protein and dietary fiber derived from yeast can be taken.

In another embodiment of the present invention, there is provided a milk-substitute composition containing a cell wall lysing enzyme-decomposition product of a yeast cell body residue, which is preferably the total amount of free glutamic acid contained by the decomposition product of less than 0.4% by mass based on the dry mass of the decomposition product. Incidentally, the terms "yeast cell body residue" and the terms "milk-substitute composition" are as mentioned above.

Also, the terms "dry mass of cell wall lysing enzyme-decomposition product of the yeast cell body residue" used in the present specification mean the mass of cell wall lysing enzyme-decomposition product of the yeast cell body residue after drying the cell wall lysing enzyme-decomposition product of the yeast cell body residue by a known method. As the drying method, it is not particularly limited and a known drying method can be used, and an atmospheric heating drying, vacuum drying, spray drying and freeze drying, etc., can be used. For example, it can be obtained by drying a sample in an atmospheric heating dryer at 105°C for 5 hours and the residue is weighed.

The cell wall lysing enzyme-decomposition product according to the present invention is characterized in that the total amount of free glutamic acid is reduced to less than a predetermined amount. Specifically, the total amount of free glutamic acid contained in the cell wall lysing enzyme-decomposition product according to the present invention is less than 0.4% by mass based on the dry mass of the decomposition product, preferably less than 0.3% by mass, and more preferably less than 0.1% by mass.

Also, the cell wall lysing enzyme-decomposition product according to the present invention is preferably one in which the total amount of not only free glutamic acid but also free amino acids is reduced to less than a predetermined amount. Specifically, the total amount of the free amino acids contained in the cell wall lysing enzyme-decomposition product according to the present invention is preferably less than 1% by mass based on the dry mass of the decomposition product, more preferably less than 0.5% by mass, and further preferably less than 0.3% by mass.

Further, in the cell wall lysing enzyme-decomposition product according to the present invention, the total amount of phenylalanine, tyrosine, arginine, isoleucine, leucine, valine, methionine and lysine, which are free amino acids known to have a bitter taste, is preferably less than 0.1% by mass based on the dry mass of the decomposition product, more preferably less than 0.07% by mass, and further preferably less than 0.05% by mass. Moreover, the total amount of alanine and proline, which are free amino acids known to have a sweet taste, is preferably less than 0.2% by mass based on the dry mass of the decomposition product, more preferably less than 0.1% by mass, and further preferably less than 0.05% by mass. In the cell wall lysing enzyme-decomposition product according to the present invention, by controlling the total amount of the free amino acids and the total amount of the free amino acids known to exhibit a bitter taste or a sweet taste, further reduction of the characteristic flavor peculiar to yeast can be expected.

The cell wall lysing enzyme-decomposition product according to the present invention is also preferably one in which the total amount of the acid components is reduced to less than a predetermined amount. Specifically, the acid components contained in the cell wall lysing enzyme-decomposition product according to the present invention are typically phosphoric acid and citric acid, and the content of phosphoric acid is preferably less than 0.20% by mass based on the dry mass of the decomposition product, more preferably less than 0.15% by mass, and further preferably less than 0.10% by mass. Also, the content of citric acid is preferably less than 0.02% by mass based on the dry mass of the decomposition product, and more preferably less than 0.01% by mass. In the cell wall lysing enzyme-decomposition product according to the present invention, by controlling the total amount of the acid components, further reduction of the characteristic flavor peculiar to yeast can be expected.

The terms "cell wall lysing enzyme" used in the present specification mean an enzyme or a combination of enzymes capable of decomposing part or all of the cell wall of the yeast. The cell wall lysing enzyme is preferably those having endo activity, and more preferably one having only endo activity. As the cell wall lysing enzyme, those having low, little or no protease activity (that is, having no protease activity) are preferred. Alternatively, when those having protease activity is used as a cell wall lysing enzyme, or when an enzyme having protease activity coexists, it is preferred to use it under conditions (for example, pH, temperature, etc.) where protease activity is suppressed.

The cell wall lysing enzymes may be, for example, those derived from a natural product, those commercially available, or those obtained by a method using genetic recombination technology, etc.

As the cell wall lysing enzymes, whereas it is not limited to these, for example, there may be mentioned glucanases and mannanases, etc. The glucanase (preferably glucanase having endo activity (preferably having endo activity alone), and/or having no protease activity) is, for example, a glucanase having β-1,3, β-1,4, and/or β-1,6 activity, preferably a glucanase derived from the genus *Streptomyces* or the genus *Talaromyces,* more preferably a glucanase derived from the genus *Streptomyces,* further preferably a glucanase derived from *Streptomyces* and having β-1,3, β-1,4, and/or β-1,6 activity, more further preferably a glucanase derived from *Streptomyces* and having β-1,3, β-1,4, and/or β-1,6 activity, and having endo activity (preferably having endo activity alone), still further preferably a glucanase derived from *Streptomyces* and having β-1,3, β-1,4, and/or β-1,6 activity, having endo activity, and having no protease activity (for example, DENAZYME GEL-L1/R, etc., available from Nagase Viita Co., Ltd.), and still further preferably a glucanase derived from *Streptomyces,* having β-1,3, β-1,4, and/or β-1,6 activity, having endo activity alone, and having no protease activity.

In one embodiment of the present invention, the cell wall lysing enzyme is a glucanase.

In one embodiment of the present invention, the cell wall lysing enzyme is a glucanase having β-1,3, β-1,4, and/or β-1,6 activity.

In one embodiment of the present invention, the cell wall lysing enzyme is a glucanase derived from *Streptomyces.*

In one preferred embodiment of the present invention, the cell wall lysing enzyme is a glucanase derived from *Streptomyces,* and having β-1,3, β-1,4, and/or β-1,6 activity.

In one embodiment of the present invention, the cell wall lysing enzyme is a glucanase having endo activity (preferably having endo activity alone).

In one preferred embodiment of the present invention, the cell wall lysing enzyme is a glucanase derived from *Streptomyces,* having β-1,3, β-1,4, and/or β-1,6 activity, and having endo activity (preferably having endo activity alone).

In one embodiment of the present invention, the cell wall lysing enzyme is a glucanase having no protease activity.

In one preferred embodiment of the present invention, the cell wall lysing enzyme is a glucanase derived from *Streptomyces,* having β-1,3, β-1,4, and/or β-1,6 activity, having endo activity (preferably having endo activity alone), and having no protease activity.

The terms "cell wall lysing enzyme-decomposition product" used in the present specification mean a product obtained by decomposing the yeast cell body residue with a cell wall lysing enzyme, specifically, a product obtained by decomposing the cell wall of the yeast contained in the yeast cell body residue with a cell wall lysing enzyme.

The decomposition product of the yeast cell body residue by a cell wall lysing enzyme is constituted by protein, lipids, ash, and dietary fiber, etc. The contained ratio of the protein is, for example, from 20% by mass to 60% by mass based on the dry mass of cell wall lysing enzyme-decomposition product of the yeast cell body residue. The contained ratio of the lipids is, for example, from 1% by mass to 10% by mass based on the dry mass of cell wall lysing enzyme-decomposition product of the yeast cell body residue. The contained ratio of the ash is, for example, from 0% by mass to 10% by mass based on the dry mass of cell wall lysing enzyme-decomposition product of the yeast cell body residue. The contained ratio of the dietary fiber is, for example, from 10% by mass to 60% by mass based on the dry mass of cell wall lysing enzyme-decomposition product of the yeast cell body residue. In addition to the general nutritional components, components which are reported to have physiological functions, such as β-glucan and α-mannan are contained in cell wall lysing enzyme-decomposition product of the yeast cell body residue. β-Glucan is a polysaccharide in which D-glucose is polymerized by β-1,3 bond and β-1,6 bond, and is classified as a dietary fiber since it is a difficultly digestible component in foods that cannot be digested by human digestive enzymes. α-Mannan is a polysaccharide in which D-mannose is polymerized by α-1,6 bond, α-1,2 bond or α-1,3 bond, and is classified as a dietary fiber since it is a difficultly digestible component in foods that cannot be digested by human digestive enzymes. The contained ratio of the β-glucan varies depending on the exo activity of the glucanase contained in the cell wall lysing enzyme and is, for example, from 0% by mass to 40% by mass based on the dry mass of cell wall lysing enzyme-decomposition product of the yeast cell body residue. The contained ratio of the α-mannan is, for example, from 10% by mass to 40% by mass based on the dry mass of cell wall lysing enzyme-decomposition product of the yeast cell body residue. The content of each component can be determined by known analytical method as mentioned above.

As the method for obtaining the "cell wall lysing enzyme-decomposition product," whereas it is not limited to these, there may be mentioned, for example, those methods known to those skilled in the art, those described in Examples, or the method containing the following:

> (a) treating the yeast cell body residue with a cell wall lysing enzyme, and
> (b) collecting the treated product obtained in (a),

or a method similar thereto.

In another embodiment of the present invention,

the decomposition product is a material obtained by the method which contains the following:

> (a) treating the yeast cell body residue with a cell wall lysing enzyme (preferably glucanase) at about 40 to about 60°C for about 1 to about 24 hours, and
> (b) collecting the treated product obtained in (a).

The terms "yeast cell body residue" in the above-mentioned (a) are as mentioned above, and are preferably the residue (water insoluble fraction) of the yeast cell body after hot water extraction of the yeast, and the yeast cell body residue in which, the soluble solid content is preferably less than 5% by mass based on its dry mass is used.

The terms "cell wall lysing enzyme" in the above-mentioned (a) are as mentioned above, and preferably are glucanase.

The term "treatment" in the above-mentioned (a) is not particularly limited as long as the conditions are such that the cell wall lysing enzyme is able to decompose the cell wall of the yeast contained in the yeast cell body residue, and can be appropriately modified according to the origin of the cell wall of the yeast, the kind and/or amount of the cell wall lysing enzyme, desired characteristics, etc. The treatment is usually carried out in the desired solvent (for example, water). For example, it is carried out using a suspension in which the yeast cell body residue is suspended in a solvent (for example, water). The suspension may be applied to sterilization treatment (for example, heat sterilization, filtration sterilization, etc.), if necessary. The treatment of the yeast cell body residue with the cell wall lysing enzyme can be carried out, for example, at a temperature of higher than 0 to lower than about 100°C (preferably about 10 to about 70°C, more preferably about 25 to about 65°C, and further preferably about 40 to about 60°C) for about 0.5 to about 120 hours (preferably about 0.5 to about 60 hours, more preferably about 1 to about 24 hours, further preferably about 3 hours to about 24 hours, and more further preferably about 12 to about 24 hours) at a pH of about 1 to about 12 (preferably about 2 to about 10, more preferably about 3 to about 8, and further preferably about 4 to about 6).

After the above-mentioned treatment, the cell wall lysing enzyme may be inactivated by high temperature treatment, acid or alkali treatment, etc., if necessary.

The treated product obtained in (a), in the above-mentioned (b), may be used in its entirety, including the residue (insoluble fraction), as the "cell wall lysing enzyme-decomposition product of the yeast cell body residue", or if necessary, those obtained by further subjecting to purification (for example, HPLC, ultrafiltration, etc.), condensation (for example, air-drying, reduced pressure filtration, etc.), sterilization (for example, heat sterilization, filtration sterilization,, etc.), drying (for example, air-drying, heat, reduced pressure, spray drying, freeze drying, etc.), etc., may be used as the "cell wall lysing enzyme-decomposition product of the yeast cell body residue". Incidentally, the conditions for these processes can be adjusted by a person skilled in the art.

The treated product obtained in (a), in the above-mentioned (b), may be used in its entirety, including the residue (insoluble fraction), as the milk-substitute composition of the present invention. Alternatively, the milk-substitute composition containing the cell wall lysing enzyme-decomposition product of the yeast cell body residue of the present invention may be in the form of a cloudy liquid or paste containing an appropriate concentration of the decomposition product in a suitable medium (preferably water) containing, for example, about 0.001 to about 30% by mass, and preferably about 5 to about 20% by mass on a dry mass basis. Alternatively, the milk-substitute composition of the present invention may be the decomposition product itself, and by utilizing the composition of the present invention, nutrients such as protein and dietary fiber derived from yeast can be taken.

The yeast cell body residue or its cell wall lysing enzyme-decomposition product mentioned above can be used in food and drink containing milk (cow's milk), or in place of the milk. For example, the "milk-substitute composition" of the present invention may be formulated in food and drink, etc., or may be mixed with food and drink before, during and/or after cooking.

In one embodiment of the present invention, there is provided a milk-substitute food and drink containing the yeast cell body residue or its cell wall lysing enzyme-decomposition product of the present invention, and oils and fats and/or sugars. A formulated amount of the yeast cell body residue or its cell wall lysing enzyme-decomposition product in the milk-substitute food and drink of the present invention may vary depending on the form of the food and drink so that it is not particularly limited, and can be made approximately 1 to 30% by mass as the dry mass of the yeast cell body residue or its cell wall lysing enzyme-decomposition product based on the mass of the milk-substitute food and drink. The milk-substitute food and drink of the present invention may be applied for drink as yeast milk (Yeast based milk), or used in cooking as a milk substitute. The milk-substitute food and drink of the present invention becomes a novel milk-substitute food and drink comprising yeast as a main raw material, which comprises protein as a main component and containing a moderate amount of oils and fats, and exhibits a milk-like appearance. If no raw material derived from animal is used as the raw material, it can be food and drink for vegetarians and vegans who avoid intake of animal foods. When oils and fats produced by using microorganisms as a raw material are used, it becomes a novel milk-substitute food and drink without using animals and plants.

A formulated amount of the yeast cell body residue or its cell wall lysing enzyme-decomposition product when applied for drink as yeast milk is not particularly limited, and it can be made approximately 1 to 30% by mass as the dry mass of the yeast cell body residue or its cell wall lysing enzyme-decomposition product based on the mass of the yeast milk, preferably 2 to 20% by mass, and further preferably 5 to 10% by mass. The yeast cell body residue or its cell wall lysing enzyme-decomposition product in the yeast milk is formulated with much amount, intake of much amount of protein derived from yeast, dietary fiber and other nutrients, etc., can be efficiently carried out. On the other hand, if the formulated amount increases, the viscosity of yeast milk increases, but the formulated amount of the yeast cell body residue or its cell wall lysing enzyme-decomposition product is within the above-mentioned range, it can be made into a drink that efficiently provides protein derived from yeast, dietary fiber and other nutrients, etc., while maintaining the preferred palatability as yeast milk.

When it is used in cooking as a milk substitute, for example, it can be used in foods such as gratins, confectionery, etc.

The terms "oils and fats" used in the present specification may be mentioned, whereas it is not limited to these, vegetable oils and fats, animal oils and fats, processed oils and fats, for example, edible safflower oil, edible grape oil, edible soybean oil, edible sunflower oil, edible corn oil, edible cottonseed oil, sesame oil, edible rapeseed oil, edible rice bran oil, edible peanut oil, edible olive oil, edible palm oil, edible palm olein, edible palm stearin, edible palm kernel oil, edible coconut oil, edible blended oil, flavored edible oil, beef tallow, lard, chicken oil, fish oil, milk fat, hydrogenated oils and fats, oils and fats produced by microorganisms such as yeast, etc., and the like.

An amount of the oils and fats contained in the milk-substitute food and drink of the present invention may vary depending on the form of the food and drink so that it is not particularly limited, and it is approximately 0.1 to 30% by mass based on the mass of the milk-substitute food and drink.

An amount of the oils and fats when applied for drink as yeast milk can be made approximately 0.1 to 30% by mass based on the mass of the yeast milk, preferably 1 to 10% by mass, and more preferably 2 to 5% by mass. In the yeast milk, when oils and fats are formulated with much amount, a richer, more satisfying, and smoother drinking experience can be obtained. On the other hand, if the formulated amount is too much, there are unpleasant effects of reducing the drinkability as the yeast milk and increasing the energy intake, but the formulated amount of oils and fats is within the above-mentioned range, it can be made a drink that is easy to drink and carrying out moderate energy intake, while providing a richer, more satisfying, and smoother drinking experience to yeast milk.

When it is applied for drink as the yeast milk, in addition to the yeast cell body residue or its cell wall lysing enzyme-decomposition product and oils and fats, an appropriate medium (preferably water) can be formulated. A formulated amount of the medium (preferably water) in the yeast milk can be made approximately 30 to 97% by mass based on the mass of the yeast milk, preferably 40 to 95% by mass, and more preferably 45 to 93% by mass. In the yeast milk, if the formulated amount of the medium (preferably water) is within the above-mentioned range, it can be made a drink with a good mouthfeel in which the yeast cell body residue or its cell wall lysing enzyme-decomposition product and oils and fats is homogeneously dispersed.

The term "carbohydrates" used in the present specification may be mentioned, for example, monosaccharides, disaccharides, trisaccharides, tetrasaccharides, oligosaccharides, polysaccharides, starch decomposition product, reduced starch decomposition product, etc., these may be used alone or in combination of two or more kinds. As the carbohydrates, whereas it is not limited to these, there may be mentioned, for example, ketotriose, aldotriose, erythrulose, ribulose, xylulose, lyxose, deoxyribose, psicose, fructose, sorbose, tagatose, allose, altrose, glucose, mannose, gulose, idose, galactose, talose, fucose, fuculose, rhamnose, sedoheptulose, sucrose, lactose, maltose, trehalose, turanose, cellobiose, raffinose, maltotriose, acarbose, stachyose, fructo-oligosaccharides, galacto-oligosaccharides, mannan oligosaccharides, glycogen, starch, cellulose, dextrin, corn syrup, corn syrup solid, glucan, etc., and these may be used alone or in combination of two or more kinds.

The milk-substitute food and drink of the present invention may further contain, whereas it is not limited to these, for example, additives such as excipients, lubricants, binders, disintegrants, pH adjusters, solvents, solubilizers, suspending agents, buffers, preservatives, antioxidants, colorants, sweeteners, surfactants, flavoring agents, etc. These additives can be used, for example, those conventionally known, and the amount to be used, etc., can be appropriately adjusted by those skilled in the art according to the purpose.

Alternatively, as the food and drink to which the "milk-substitute composition" of the present invention can be applied or contains, whereas it is not limited to these, there may be mentioned, for example, drink and food which are contained or are planning to be contained milk (for example, cow's milk, soy milk, almond milk, all milk, coconut milk, etc.) or milk powder, including, for example, soups, dressings, semi-solid dressings, mayonnaise, confectionery, chocolate, margarine, fat spreads, ice cream, lacto-ice cream, ice milk, bread, retort foods, extract seasonings, processed oils and fats, cheese, cheese food, processed cheese, purine and other processed foods, etc.

The applied amount of the "milk-substitute composition" of the present invention is not particularly limited as long as the object of the present invention can be achieved. It can be added to the total amount of the food and drink, etc., or the processed product thereof, for example, about 0.001 to about 30% by mass, preferably about 0.01 to about 20% by mass, and more preferably about 0.1 to about 15% by mass, as the yeast cell body residue or its cell wall lysing enzyme-decomposition product (dry mass).

In another embodiment of the present invention, there is provided a method for improving the flavor of a milk-substitute composition containing a yeast cell body residue, which comprises reducing the total amount of free glutamic acid contained in the residue to less than 0.5% by mass based on the dry mass of the residue.

In another embodiment of the present invention, there is provided a method for improving the flavor of a milk-substitute composition containing a cell wall lysing enzyme-decomposition product of a yeast cell body residue, which comprises reducing the total amount of free glutamic acid contained in the decomposition product to less than 0.4% by mass based on the dry mass of the decomposition product.

In another embodiment of the present invention, there is provided use of a yeast cell body residue as a milk-substitute composition, wherein the total amount of free glutamic acid contained in the residue is less than 0.5% by mass based on a dry mass of the residue.

In another embodiment of the present invention, there is provided use of a milk-substitute composition of residue of a cell wall lysing enzyme-decomposition product of the yeast cell body, the total amount of free glutamic acid contained in the decomposition product is less than 0.4% by mass based on the dry mass of the decomposition product.

For these embodiments of the present invention, the explanation given above for the embodiments of a milk-substitute composition containing the yeast cell body residue or its cell wall lysing enzyme-decomposition product can be applied in the same manner.

EXAMPLES

Hereinafter, the present invention will be explained in more detail by referring to Examples, but these Examples do not intend to limit the scope of the present invention.

[Comparative Example 1]

Dried yeast (Hyper Yeast HG-DY available from Asahi Group Foods, Ltd.) was extracted with hot water, and heavy phase (insoluble fraction) obtained by separating from yeast extract in a nozzle-type continuous centrifuge was sterilized at 125°C for 40 seconds and spray dried to obtain a yeast cell body residue as a composition of Comparative Example 1.

[Comparative Example 2]

A slurry (solid content of 16%) in which the yeast cell body residue obtained in Comparative Example 1 was suspended in deionized water was sterilized in an autoclave (121°C for 20 minutes), and 0.2% by mass of glucanase (DENAZYME GEL-L1/R, available from Nagase Viita Co., Ltd.) based on the dry mass of the yeast cell body residue was added under the conditions of 50°C and pH 5.3, and treated at 50°C for 18 hours. Thereafter, the treated product was heated to about 80°C to deactivate the glucanase, and the treated product was concentrated under reduced pressure using an evaporator to a concentration of 30% by mass, and spray dried to obtain a cell wall lysing enzyme-decomposition product as a composition of Comparative Example 2.

These compositions of Comparative Examples 1 and 2 were prepared in accordance with Example 1 of Patent Document 1.

[Example 1]

A slurry (solid content of 16%) in which the yeast cell body residue obtained in Comparative Example 1 was suspended by water was prepared. Three nozzle-type continuous centrifuges were arranged in series and solid-liquid separation was carried out while adding water to obtain a heavy phase from which soluble components were removed. The obtained heavy phase was sterilized with the conditions of 125°C for 40 seconds, and the yeast cell body residue was obtained as a composition of Example 1 by spray drying.

[Example 2]

A heavy phase after sterilization prepared in the same manner as in Example 1 was adjusted to the conditions of 50°C and pH 5.3, and treated by adding thereto 0.2% by mass of glucanase (DENAZYME GEL-L1/R, available from Nagase Viita Co., Ltd.) based on the dry mass at 50°C for 18 hours. The treated product was treated with the conditions of 125°C for 40 seconds to sterilize and simultaneously inactivate the glucanase, and a cell wall lysing enzyme-decomposition product was obtained as a composition of Example 2 by spray drying.

[Test Example 1]

The raw materials were mixed in accordance with the composition shown in Table 1, emulsified in a homogenizer (Homogenizer LAB 1000, SMT CO., LTD.: 20 MPa, twice), and sterilized (121°C for 20 minutes) in an autoclave to obtain Formulation 1 and Formulation 2 of the milk-substitute composition. It was confirmed that both Formulation 1 and Formulation 2 maintained a cloudy, emulsified, milk-like appearance for a certain period of time (3 days) or longer at room temperature. Incidentally, Formulation 1 was prepared in the same manner as in Formulation 23 of Patent document 1.

[Table 1]

| Composition | Formulation 1 | Formulation 2 |
|---|---|---|
| Edible sunflower oil | 3.8g | 3.8g |
| Comparative Example 2 | 8.8g | - |
| Example 2 | - | 8.8g |
| Water | 87.4g | 87.4g |
| Total | 100g | 100g |

[Sensory evaluation of Formulation 1 and Formulation 2]

Sensory evaluation was carried out with regard to Formulation 1 and Formulation 2 as follows. Sensory evaluation of each Formulation cooled to 4°C was carried out by five expert panelists. With regard to evaluation items "umami," "sourness," "milk-like flavor" and "palatability," Formulation 1 was evaluated to as 0 point as control, the strength of the flavor of the test samples was evaluated based on the following evaluation criteria. Incidentally, it can be considered that "umami" and "sourness" are preferred to be negative value (to be weak as compared with the control), and "milk-like flavor" and "palatability" are preferred to be positive value. Also, here, "milk-like flavor" was evaluated based on its overall similarity to animal milk in terms of a faint sweet aroma after swallowing, mildness, absence of off-flavor and odor, and plainness.

[Evaluation criteria]

-3 point: Extremely weak
-2 point: Weak
-1 point: Slightly weak
0 point: Control
+1 point: Slightly strong
+2 point: Strong
+3 point: Extremely strong

[Results]

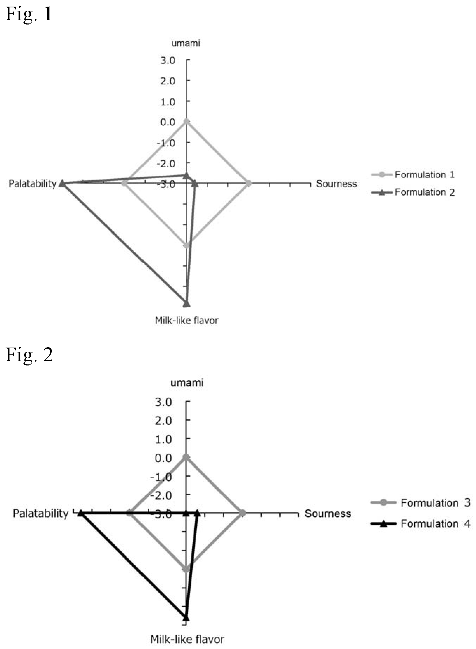

The evaluation results are shown in Fig. 1 as average values. From these results, Formulation 2 obtained in Test Example 1 was reduced in "umami" and "sourness" as compared with Formulation 1, and "milk-like flavor" and "palatability" were increased. It could be understood that Formulation 2 clearly exhibited desirable sensory characteristics as a milk-substitute composition as compared with Formulation 1.

[Test Example 2]

The raw materials were mixed in accordance with the composition shown in Table 2 to obtain Formulation 3 and Formulation 4. Further, after mixing the raw materials, they were emulsified by a homogenizer (Homogenizer LAB1000, SMT CO., LTD.: 20 MPa, 2 times) and sterilized (121°C for 20 minutes) in an autoclave to obtain Formulations 5 to 12 of milk-substitute compositions containing yeast compositions and oils in different ratios. It was confirmed that all of Formulations 5 to 12 maintained a cloudy, emulsified, milk-like appearance for a certain period of time (3 days) or longer at room temperature.

[Table 2]

| | Formulation 3 | Formulation 4 | Formulation 5 | Formulation 6 | Formulation 7 | Formulation 8 | Formulation 9 | Formulation 10 | Formulation 11 | Formulation 12 |
|---|---|---|---|---|---|---|---|---|---|---|
| Comparative Example 2 | 5 g | - | 5 g | - | 5 g | - | 20 g | - | 20 g | - |
| Example 2 | - | 5 g | - | 5 g | - | 5 g | - | 20 g | - | 20 g |
| Sunflower oil | - | | 2 g | 2 g | 30 g | 30 g | 2 g | 2 g | 30 g | 30 g |
| Water | 95 g | 95 g | 93 g | 93 g | 65 g | 65 g | 78 g | 78 g | 50 g | 50 g |
| Total | 100 g | 100 g | 100 g | 100 g | 100 g | 100 g | 100 g | 100 g | 100 g | 100 g |

[Sensory evaluation of Formulation 3 to Formulation 12]

Sensory evaluation was carried out with regard to the obtained Formulation 3 to 12 as follows. Sensory evaluation of each Formulation cooled to 4°C was carried out by five expert panelists. Formulation 3 was compared with Formulation 4, Formulation 5 with Formulation 6, Formulation 7 with Formulation 8, Formulation 9 with Formulation 10, and Formulation 11 with Formulation 12, and Formulation 3, Formulation 5, Formulation 7, Formulation 9 and Formulation 11 were used as controls, respectively. With regard to evaluation items "umami," "sourness," "milk-like flavor" and "palatability," the control was evaluated to as 0 point, and the strength of the flavor of the test samples was evaluated based on the above-mentioned evaluation criteria.

[Results]

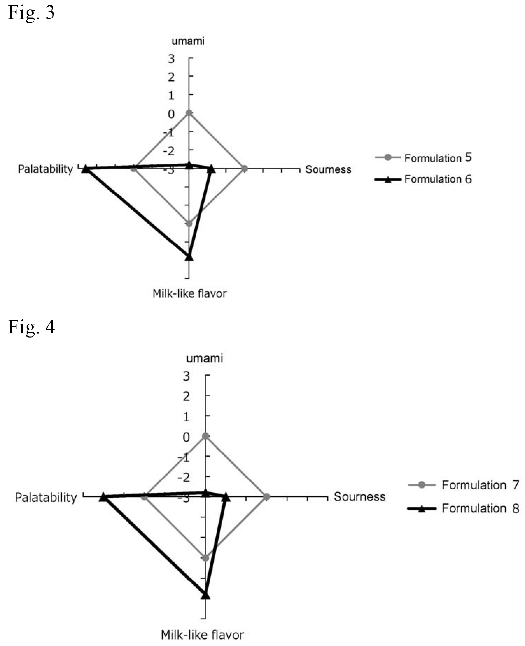

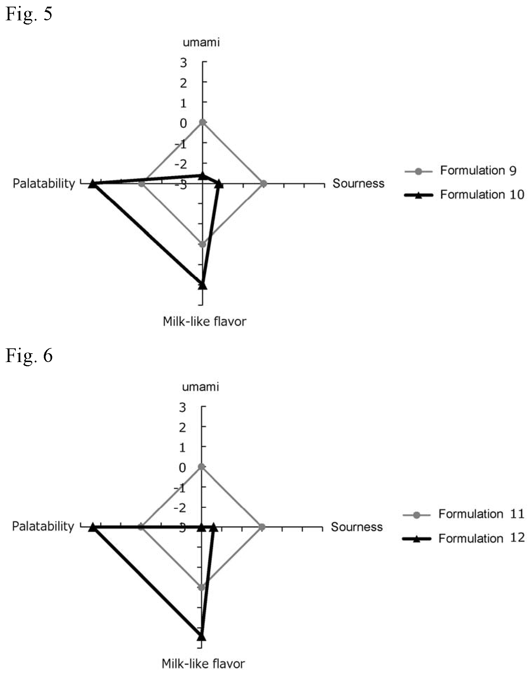

The results of the evaluation by 5 expert panelists are shown in Fig. 2 to Fig. 6 as average values. From this results, Formulation 4 is reduced in "umami" and "sourness" and increased in "milk-like flavor" and "palatability" as compared with Formulation 3, whereby it showed preferable sensory characteristics as a milk-substitute composition. Formulation 6 compared with Formulation 5, Formulation 8 compared with Formulation 7, Formulation 10 compared with Formulation 9, and Formulation 12 compared with Formulation 11, they similarly showed reduced in "umami" and "sourness", and increased in "milk-like flavor" and "palatability," whereby they showed preferable sensory characteristics as a milk-substitute composition. The composition of Example 2 showed more favorable flavor than the composition of Comparative Example 2 in a wide range of concentrations (from 5% to 20%) and in a wide range of oils and fats concentrations (from 0% to 30%).

[Comparative Examples 3 and 4 and Example 3]

The yeast cell body residue obtained in Comparative Example 1 was suspended in water to prepare a slurry (solid content of 16%). Solid-liquid separation was carried out by treating in a centrifuge (Avanti J-26XP, manufactured by Beckman Coulter Inc.) at 5,000 × g for 5 minutes, and the supernatant was removed. The operation in which the same amount of water as the supernatant was added to the residue to suspend it and solid-liquid separation was carried out once again to collect the insoluble fraction. The obtained insoluble fraction was sterilized with the conditions of 121°C for 20 minutes, and lyophilized to obtain the yeast cell body residue of Example 3. In Example 3, in the same manner except that the amount of water added after the first solid-liquid separation was reduced, the yeast cell body residue in which the degree of washing was different were obtained as compositions of Comparative Examples 3 and 4.

[Comparative Examples 5 and 6 and Example 4]

Next, in Comparative Examples 3 and 4 and Example 3, the insoluble fraction after sterilization was prepared as a slurry (solid content of 16%) with the conditions of 50°C and pH 5.3, and 0.2% by mass of glucanase (DENAZYME GEL-L1/R, available from Nagase Viita Co., Ltd.) was added based on the dry mass and treated at 50°C for 18 hours. Thereafter, the treated product was then treated at the conditions of 121°C for 20 minutes to sterilize and simultaneously inactivate the glucanase, and freeze-dried to obtain decomposition products by a cell wall lysing enzyme in which the degree of washing was different as compositions of Comparative Examples 5 and 6 and Example 4.

[Test Example 3]

With regard to each composition obtained in Examples 1 to 4 and Comparative Examples 1 to 6, the content of the free amino acids, the content of phosphoric acid and the content of citric acid were measured.

[Free amino acid analysis]

The content of free amino acids was measured by the AccQ-Tag Ultra labeling method using an Acquity UPLC analyzer (manufactured by Waters Corp., USA). Calibration curves were prepared using amino acid mixture standard solution Type H (available from FUJIFILM Wako Pure Chemical Corporation), L-asparagine monohydrate (available from FUJIFILM Wako Pure Chemical Corporation), L(+)-glutamine (available from FUJIFILM Wako Pure Chemical Corporation), L-tryptophan (available from FUJIFILM Wako Pure Chemical Corporation) and γ-aminobutyric acid (available from Sigma-Aldrich Japan, LLC). By the method, the free amino acids in the samples were selectively quantified and the content thereof (% by mass) was calculated. Incidentally, total free amino acids mean the sum of the content of each free amino acid per dry mass of the yeast cell body residue or the cell wall lysing enzyme-decomposition product. The content of the amino acids (% by mass) per dry mass of the yeast cell body residue (Comparative Examples 1, 3 and 4 and Examples 1 and 3) is shown in Table 3. Similarly, the content of the amino acids (% by mass) per dry mass of the cell wall lysing enzyme-decomposition product (Comparative Examples 2, 5 and 6 and Examples 2 and 4) is shown in Table 4.

[Table 3]

| | His | Asn | Ser | Gln | Arg | Gly | Asp | Glu | Thr | Ala | GABA |
|---|---|---|---|---|---|---|---|---|---|---|---|
| Comparative Example 1 | 0.00 | 0.01 | 0.01 | 0.00 | 0.02 | 0.03 | 0.05 | 0.77 | 0.00 | 0.11 | 0.00 |
| Comparative Example 3 | 0.00 | 0.01 | 0.01 | 0.00 | 0.01 | 0.03 | 0.05 | 0.69 | 0.00 | 0.10 | 0.00 |
| Comparative Example 4 | 0.00 | 0.01 | 0.01 | 0.00 | 0.01 | 0.02 | 0.04 | 0.51 | 0.00 | 0.07 | 0.00 |
| Example 1 | 0.00 | 0.00 | 0.00 | 0.00 | 0.00 | 0.01 | 0.01 | 0.07 | 0.00 | 0.03 | 0.00 |
| Example 3 | 0.00 | 0.01 | 0.01 | 0.00 | 0.01 | 0.02 | 0.02 | 0.29 | 0.00 | 0.05 | 0.00 |
| | Pro | Cys | Lys | Tyr | Met | Val | Ile | Leu | Phe | Trp | Total free amino acids |
| Comparative Example 1 | 0.11 | 0.00 | 0.01 | 0.02 | 0.00 | 0.02 | 0.01 | 0.01 | 0.01 | 0.00 | 1.22 |
| Comparative Example 3 | 0.09 | 0.00 | 0.01 | 0.02 | 0.00 | 0.02 | 0.01 | 0.01 | 0.01 | 0.00 | 1.09 |
| Comparative Example 4 | 0.07 | 0.00 | 0.01 | 0.01 | 0.00 | 0.01 | 0.01 | 0.01 | 0.00 | 0.00 | 0.80 |
| Example 1 | 0.01 | 0.00 | 0.00 | 0.00 | 0.00 | 0.00 | 0.00 | 0.00 | 0.00 | 0.00 | 0.14 |
| Example 3 | 0.05 | 0.00 | 0.00 | 0.01 | 0.00 | 0.01 | 0.01 | 0.01 | 0.00 | 0.00 | 0.48 |

[Table 4]

| | His | Asn | Ser | Gln | Arg | Gly | Asp | Glu | Thr | Ala | GABA |
|---|---|---|---|---|---|---|---|---|---|---|---|
| Comparative Example 2 | 0.00 | 0.02 | 0.01 | 0.00 | 0.02 | 0.04 | 0.06 | 0.72 | 0.00 | 0.12 | 0.00 |
| Comparative Example 5 | 0.00 | 0.01 | 0.01 | 0.00 | 0.02 | 0.03 | 0.05 | 0.58 | 0.00 | 0.10 | 0.00 |
| Comparative Example 6 | 0.00 | 0.01 | 0.01 | 0.00 | 0.02 | 0.02 | 0.04 | 0.45 | 0.00 | 0.07 | 0.00 |
| Example 2 | 0.00 | 0.00 | 0.00 | 0.00 | 0.00 | 0.01 | 0.00 | 0.06 | 0.00 | 0.02 | 0.00 |
| Example 4 | 0.00 | 0.00 | 0.01 | 0.00 | 0.02 | 0.02 | 0.02 | 0.24 | 0.00 | 0.05 | 0.00 |
| | Pro | Cys | Lys | Tyr | Met | Val | Ile | Leu | Phe | Trp | Total free amino acids |
| Comparative Example 2 | 0.12 | 0.00 | 0.01 | 0.02 | 0.00 | 0.02 | 0.02 | 0.01 | 0.01 | 0.00 | 1.21 |
| Comparative Example 5 | 0.10 | 0.00 | 0.01 | 0.02 | 0.00 | 0.02 | 0.01 | 0.01 | 0.01 | 0.00 | 1.00 |
| Comparative Example 6 | 0.07 | 0.00 | 0.01 | 0.01 | 0.00 | 0.01 | 0.01 | 0.01 | 0.01 | 0.00 | 0.76 |
| Example 2 | 0.01 | 0.00 | 0.00 | 0.00 | 0.00 | 0.00 | 0.00 | 0.00 | 0.00 | 0.00 | 0.13 |
| Example 4 | 0.05 | 0.00 | 0.01 | 0.01 | 0.00 | 0.01 | 0.01 | 0.01 | 0.00 | 0.00 | 0.45 |

[Acid component analyses]

The content of the acid components was measured using Prominence HPLC organic acid analysis system (manufactured by Shimadzu Corporation, Japan). Each composition of Examples and Comparative Examples was prepared as a 5% by mass suspension by deionized water, and the supernatant obtained by centrifugation and filtered through a filter with a pore diameter of 0.45 $\mu$m was analyzed as the sample solution for measurement. As the objects to be analyzed, phosphoric acid, citric acid, pyruvic acid, malic acid, succinic acid, lactic acid, acetic acid, pyroglutamic acid and formic acid were measured, and excluding acid components with very low concentrations, phosphoric acid and citric acid were determined. The content of the acid components (% by mass) per dry mass of the yeast cell body residue (Comparative Examples 1, 3 and 4, and Examples 1 and 3), calculated from the obtained measured values, is shown in Table 5. Similarly, the content of the acid components (% by mass) per dry mass of the decomposition products by a cell wall lysing enzyme (Comparative Examples 2, 5 and 6 and Examples 2 and 4) is shown in Table 6.

[Table 5]

|  | Phosphoric acid | Citric acid |
|---|---|---|
| Comparative Example 1 | 0.124 | 0.035 |
| Comparative Example 3 | 0.100 | 0.027 |
| Comparative Example 4 | 0.073 | 0.022 |
| Example 1 | 0.017 | 0.000 |
| Example 3 | 0.046 | 0.011 |

[Table 6]

|  | Phosphoric acid | Citric acid |
|---|---|---|
| Comparative Example 2 | 0.180 | 0.028 |
| Comparative Example 5 | 0.194 | 0.020 |
| Comparative Example 6 | 0.167 | 0.019 |
| Example 2 | 0.011 | 0.000 |
| Example 4 | 0.109 | 0.000 |

[Test Example 4]

With regard to each composition of the obtained yeast cell body residue (Comparative Examples 1, 3 and 4 and Examples 1 and 3), the content ratios of soluble solids were measured.

[Measurement of content ratio of contained soluble solids]

The ratio (% by mass) of the solid content which is soluble in water contained in the yeast cell body residue to the solid content of the residue was calculated by the following formula (1) by preparing a 10% by mass suspension of each of the compositions of Examples and Comparative Examples by deionized water.

Content ratio of soluble solids = Concentration B of solid component/Concentration A of solid component x 100 · · · Formula (1)    Formula (1)

In the above-mentioned formula (1), "Concentration A of solid component" indicates % by mass of a dried product obtained by drying X g of the subject sample at 105°C for 5 hours, and "Concentration B of solid component" indicates % by mass of a dried product obtained by centrifuging X g of the subject sample at 5,000 × g for 5 minutes and drying the obtained supernatant at 105°C for 5 hours. The content ratio of soluble solids calculated by formula (1) are shown in Table 7.

[Table 7]

|  | Soluble solid content ratio (% by mass) |
|---|---|
| Comparative Example 1 | 12. 39 |
| Comparative Example 3 | 9. 74 |
| Comparative Example 4 | 7. 39 |
| Example 1 | 3. 52 |

(continued)

| | Soluble solid content ratio (% by mass) |
|---|---|
| Example 3 | 4. 57 |

[Test Example 5]

[Sensory evaluation of yeast cell body residue]

With regard to each composition obtained in Comparative Examples 1, 3 and 4 and Examples 1 and 3, sensory evaluation was carried out as follows. First, each raw material was added to water at normal temperature to reach 5 % by mass, stirred well, and then sensory evaluation was carried out by five expert panelists. With regard to evaluation items "umami," "sourness," "milk-like flavor" and "palatability," the composition of Comparative Example 1 was evaluated to as 0 point as control, and the strength of the flavor of each sample was evaluated based on the above-mentioned evaluation criteria.

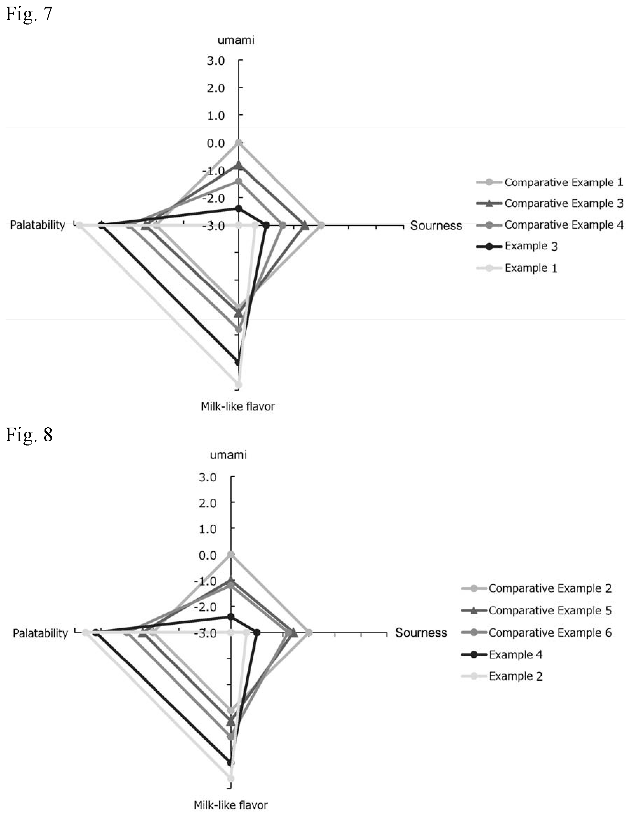

The sensory evaluation results are shown in Fig. 7 as average values. Compared to the composition of Comparative Example 1, the compositions of Comparative Examples 3 and 4 also showed a trend toward a reduction in "umami" and "sourness" and an increase in "milk-like flavor" and "palatability". On the other hand, in the compositions of Example 1 and 3, "umami" and "sourness" were reduced to a score of -2 or less, and "milk-like flavor" and "palatability" increased to a score of +2 or more, which showed particularly preferred sensory characteristics.

As shown in the free amino acids analysis in the above-mentioned Table 3, the content of the free amino acids of the compositions of Comparative Examples 3 and 4 and Examples 1 and 3 was reduced from that of the composition of Comparative Example 1, reflecting the degree of washing in the production process. In the compositions of Examples 1 and 3, which exhibited particularly preferred sensory characteristics, the content of the total free amino acids was 0.48% by mass or less, the sum of phenylalanine, tyrosine, arginine, isoleucine, leucine, valine, methionine and lysine which are free amino acids and are known to exhibit bitter taste was 0.04% by mass or less, and the content of glutamic acid that exhibit sourness and umami was 0.29% by mass or less. Further, in the compositions of Examples 1 and 3, the content of alanine and proline that exhibit sweet taste was each 0.05% by mass or less.

Also, as shown in acid component analysis in the above-mentioned Table 5, the content of phosphoric acid and citric acid of the compositions of Examples 1 and 3 that showed particularly preferred sensory characteristics was each 0.046% by mass or less and 0.011% by mass or less. Further, as shown in the content ratio of the soluble solids in the above-mentioned Table 7, the solid content which is soluble in water contained in the compositions of Examples 1 and 3 that showed particularly preferred sensory characteristics was each 3.52% by mass and 4.57% by mass, indicating that the components that exhibit flavor soluble in water were less than in Comparative Examples. From these results, it was indicated that a material that shows preferred sensory characteristics could be obtained by removing tasting components from the yeast cell body residue to a certain level or less.

[Sensory evaluation of decomposition product by cell wall lysing enzyme]

Sensory evaluation of each composition obtained in Comparative Examples 2, 5 and 6 and Examples 2 and 4 was carried out as follows. First, each raw material was added to water at normal temperature so as to reach 5% by mass, and after stirring well, sensory evaluation was carried out by five expert panelists. With regard to evaluation items "umami," "sourness," "milk-like flavor" and "palatability," the composition of Comparative Example 2 was evaluated to as 0 point as control, and the strength of the flavor of each sample was evaluated based on the above-mentioned evaluation criteria.

The sensory evaluation results are shown in Fig. 8 as average values. Compared to the composition of Comparative Example 2, the compositions of Comparative Examples 5 and 6 also showed a trend toward a reduction in "umami" and "sourness" and an increase in "milk-like flavor" and "palatability". On the other hand, in the compositions of Example 2 and 4, "umami" and "sourness" were reduced to a score of -2 or less, and "milk-like flavor" and "palatability" increased to a score of +2 or more, which showed particularly preferred sensory characteristics.

As shown in the free amino acids analysis in the above-mentioned Table 4, the content of the free amino acids of the compositions of Comparative Examples 5 and 6 and Examples 2 and 4 was reduced from that of the composition of Comparative Example 2, reflecting the degree of washing in the production process. In the compositions of Examples 2 and 4, which exhibited particularly preferred sensory characteristics, the content of the total free amino acids was 0.45% by mass or less, the sum of phenylalanine, tyrosine, arginine, isoleucine, leucine, valine, methionine and lysine which are free amino acids and are known to exhibit bitter taste was 0.06% by mass or less, and the content of glutamic acid that exhibit sourness and umami was 0.24% by mass or less. Further, in the compositions of Examples 2 and 4, the content of alanine and proline that exhibit sweet taste was each 0.05% by mass or less.

Also, as shown in the acid component analysis in the above-mentioned Table 6, the compositions of Examples 2 and 4, which exhibited particularly preferred sensory characteristics, had phosphoric acid of 0.109% by mass or less and citric acid of 0% by mass. From these results, it was indicated that a material that shows preferred sensory characteristics could be obtained by removing tasting components from the yeast cell body residue to a certain level or less.

UTILIZABILITY IN INDUSTRY

In the composition of the present invention, the total amount of free amino acids (in particular, glutamic acid) and acid components (in particular, phosphoric acid), etc., contained in the yeast cell body residue or its cell wall lysing enzyme-decomposition product to less than a specific amount, the characteristic flavor (for example, umami, sourness, etc.) peculiar to yeast is significantly reduced, so that it can be formulated, as a milk-substitute composition, in various foods and drinks at higher concentrations than those of conventional products. Accordingly, the composition of the present invention can be provided as foods and drinks in various forms, and the intake of protein derived from yeast, dietary fiber, and other nutrients can be more easily carried out through the foods and drinks.

**Claims**

1. A milk-substitute composition comprising a yeast cell body residue, wherein a total amount of free glutamic acid contained in the residue is less than 0.5% by mass based on a dry mass of the residue.

2. The composition according to claim 1, wherein a total amount of free amino acids contained in the residue is less than 1% by mass based on a dry mass of the residue.

3. The composition according to claim 1, wherein a total amount of phosphoric acid contained in the residue is less than 0.1% by mass based on a dry mass of the residue.

4. The composition according to claim 1, wherein the residue is a water-insoluble fraction after hot water extraction of yeast.

5. The composition according to claim 1, wherein a soluble solid content contained in the residue is less than 5% by mass based on a dry mass of the residue.

6. A milk-substitute composition comprising a cell wall lysing enzyme-decomposition product of a yeast cell body residue, wherein a total amount of free glutamic acid contained in the decomposition product is less than 0.4% by mass based on a dry mass of the decomposition product.

7. The composition according to claim 6, wherein a total amount of free amino acids contained in the decomposition product is less than 1% by mass based on a dry mass of the decomposition product.

8. The composition according to claim 6, wherein a total amount of phosphoric acid contained in the decomposition product is less than 0.20% by mass based on a dry mass of the decomposition product.

9. The milk-substitute composition comprising a cell wall lysing enzyme-decomposition product of a yeast cell body residue according to claim 6, wherein a soluble solid content contained in the residue is less than 5% by mass based on a dry mass of the residue.

10. The composition according to claim 6, wherein the decomposition product is a material obtained by a method which comprises the following:

    (a) treating the yeast cell body residue with a cell wall lysing enzyme at 40 to 60°C for 1 to 24 hours, and
    (b) collecting the treated product obtained in (a).

11. The composition according to claim 6, wherein the cell wall lysing enzyme is a glucanase.

12. The composition according to claim 6, wherein the cell wall lysing enzyme is a glucanase having $\beta$-1,3, $\beta$-1,4 and/or $\beta$-1,6 activity.

13. The composition according to claim 6, wherein the cell wall lysing enzyme is a glucanase derived from *Streptomyces*.

14. The composition according to claim 6, wherein the cell wall lysing enzyme is a glucanase having no protease activity.

15. A milk-substitute food and drink comprising the composition according to any of claims 1 to 14.

16. The milk-substitute food and drink according to claim 15, which further comprises from 0.1 to 30% by mass of oils and fats.

17. A method for improving flavor of a milk substitute composition comprising a yeast cell body residue, which comprises reducing a total amount of free glutamic acid contained in the residue to less than 0.5% by mass based on a dry mass of the residue.

18. A method for improving flavor of a milk substitute composition comprising a cell wall lysing enzyme-decomposition product of a yeast cell body residue, which comprises reducing a total amount of free glutamic acid contained in the decomposition product to less than 0.4% by mass based on a dry mass of the decomposition product.

19. Use of a yeast cell body residue as a milk-substitute composition, wherein a total amount of free glutamic acid contained in the residue is less than 0.5% by mass based on a dry mass of the residue.

20. Use of a cell wall lysing enzyme-decomposition product of a yeast cell body residue as a milk-substitute composition, wherein a total amount of free glutamic acid contained in the decomposition product is less than 0.4% by mass based on a dry mass of the decomposition product.

Fig. 1

Fig. 2

Fig. 3

Fig. 4

Fig. 5

Fig. 6

Fig. 7

umami

3.0
2.0
1.0
0.0
-1.0
-2.0
-3.0

Palatability

Sourness

Milk-like flavor

- Comparative Example 1
- Comparative Example 3
- Comparative Example 4
- Example 3
- Example 1

Fig. 8

umami

3.0
2.0
1.0
0.0
-1.0
-2.0
-3.0

Palatability

Sourness

Milk-like flavor

- Comparative Example 2
- Comparative Example 5
- Comparative Example 6
- Example 4
- Example 2

## INTERNATIONAL SEARCH REPORT

| | International application No. |
|---|---|
| | **PCT/JP2024/025900** |

### A. CLASSIFICATION OF SUBJECT MATTER

*A23C 11/10*(2021.01)i; *A23L 5/00*(2016.01)i; *A23L 31/15*(2016.01)i
FI:  A23C11/10; A23L5/00 J; A23L31/15

According to International Patent Classification (IPC) or to both national classification and IPC

### B. FIELDS SEARCHED

Minimum documentation searched (classification system followed by classification symbols)

A23C11/10; A23L5/00; A23L31/15

Documentation searched other than minimum documentation to the extent that such documents are included in the fields searched

Published examined utility model applications of Japan 1922-1996
Published unexamined utility model applications of Japan 1971-2024
Registered utility model specifications of Japan 1996-2024
Published registered utility model applications of Japan 1994-2024

Electronic data base consulted during the international search (name of data base and, where practicable, search terms used)

JSTPlus/JMEDPlus/JST7580 (JDreamIII)

### C. DOCUMENTS CONSIDERED TO BE RELEVANT

| Category* | Citation of document, with indication, where appropriate, of the relevant passages | Relevant to claim No. |
|---|---|---|
| A | JP 2022-135934 A (ASAHI GROUP FOODS, LTD.) 15 September 2022 (2022-09-15)<br>entire text, all drawings | 1-20 |
| A | JP 2007-252239 A (ASAHI BREWERIES, LTD.) 04 October 2007 (2007-10-04)<br>entire text, all drawings | 1-20 |
| A | JP 2018-078863 A (BUSSAN FOOD SCIENCE K.K.) 24 May 2018 (2018-05-24)<br>entire text, all drawings | 1-20 |
| A | JP 2022-074430 A (MITSUBISHI CORPORATION LIFE SCIENCES LTD.) 18 May 2022<br>(2022-05-18)<br>entire text, all drawings | 1-20 |
| A | JP 2005-245438 A (NIPPON PAPER CHEMICALS CO., LTD.) 15 September 2005<br>(2005-09-15)<br>entire text, all drawings | 1-20 |

☐ Further documents are listed in the continuation of Box C.  ☑ See patent family annex.

| | |
|---|---|
| * Special categories of cited documents: | "T" later document published after the international filing date or priority date and not in conflict with the application but cited to understand the principle or theory underlying the invention |
| "A" document defining the general state of the art which is not considered to be of particular relevance | |
| "D" document cited by the applicant in the international application | "X" document of particular relevance; the claimed invention cannot be considered novel or cannot be considered to involve an inventive step when the document is taken alone |
| "E" earlier application or patent but published on or after the international filing date | |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
|---|---|
| **17 September 2024** | **24 September 2024** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | |
| | Telephone No. |

Form PCT/ISA/210 (second sheet) (July 2022)

## INTERNATIONAL SEARCH REPORT
### Information on patent family members

International application No.

**PCT/JP2024/025900**

| Patent document cited in search report | | | Publication date (day/month/year) | Patent family member(s) | | | Publication date (day/month/year) |
|---|---|---|---|---|---|---|---|
| JP | 2022-135934 | A | 15 September 2022 | US 2024/0138456 A1 entire text, all drawings WO 2022/185762 A1 EP 4303284 A1 CN 117015312 A KR 10-2023-0152686 A | | | |
| JP | 2007-252239 | A | 04 October 2007 | (Family: none) | | | |
| JP | 2018-078863 | A | 24 May 2018 | (Family: none) | | | |
| JP | 2022-074430 | A | 18 May 2022 | (Family: none) | | | |
| JP | 2005-245438 | A | 15 September 2005 | (Family: none) | | | |

Form PCT/ISA/210 (patent family annex) (July 2022)

## EP 4 748 235 A1

**Patent documents cited in the description**

- JP 2022135934 A